# EUROPÄISCHE PATENTANMELDUNG

(11) **EP 4 180 002 A1**
(43) Veröffentlichungstag der Anmeldung: **17.05.2023**
(21) Anmeldenummer: 21020570.4
(22) Anmeldetag: 16.11.2021
(51) Int. Cl.: A61C 7/10, A61C 7/36, A61F 5/56

(54) **KIEFERORTHOPÄDISCHE APPARATUR SOWIE HERSTELLUNGSVERFAHREN**

(71) Anmelder: Dentavenir GmbH & Co. KG, 90491 Nürnberg (DE)
(72) Erfinder: Giotakis, Georgios, 90491 Nürnberg (DE); Utomo, Markus Setiono, 90408 Nürnberg (DE); Bauer, Franz Xaver, 83098 Brannenburg (DE); Czizegg, Wolfgang Christian, 82343 Pöcking (DE)

(57) **Zusammenfassung**

Die Erfindung betrifft eine der Mundhöhle eines Menschen anordenbare kieferorthopädische Apparatur (1) zur Beeinflussung der Relativposition von Ober- und Unterkiefer, insbesondere zur Behandlung von Unterkieferrücklagen. Erfindungsgemäß ist vorgesehen, dass die Protrusionsmittel (16) einteilig mit dem ersten und/oder dem zweiten Oberkieferplattenabschnitt (4, 5) als 3D-Druckteil aus Kunststoff ausgebildet sind und der mindestens eine Kontaktbereich (24, 25) einteilig mit dem ersten und/oder dem zweiten Unterkieferplattenabschnitt (8, 9) als 3D-Druckteil aus Kunststoff ausgebildet ist. Ferner betrifft die Erfindung ein zugehöriges Herstellungsverfahren.

## Beschreibung

Die Erfindung betrifft eine, insbesondere als Vorschubdoppelplatte ausgebildete, kieferorthopädische Apparatur zum lösbaren Einsetzen in der Mundhöhle eines menschlichen Patienten zur Beeinflussung der Relativposition von Ober- und Unterkiefer, insbesondere zur Behandlung von Unterkieferrücklagen gemäß dem Oberbegriff des Anspruchs 1. Bei der kieferorthopädischen Apparatur handelt es sich um ein Zwei-Plattensystem, umfassend eine am Oberkiefer innerhalb der Mundhöhle anordenbare Oberkieferplatte und einer dieser in Bezug auf die Okklusionsebene gegenüberliegende Unterkieferplatte. Jede der Platten umfasst einen ersten, insbesondere aus Sicht des Patienten in Bezug auf die Sagittalebene linken, bevorzugt halbbogenförmigen Plattenabschnitt und einen zweiten, insbesondere aus Sicht des Patienten in Bezug auf die Sagittalebene rechten, bevorzugt halbbogenförmigen Plattenabschnitt. Der erste und zweite Plattenabschnitt sowohl der Ober - als auch der Unterkieferplatte liegen, zumindest größtenteils auf unterschiedlichen Seiten der Sagittalebene und weisen seitliche, der jeweils benachbarten linken bzw. rechten Zahnbogenhälfte zugewandte Taschen zur Anlage an zum Inneren der Mundhöhle gerichteten Zahnflächen auf. Die kieferorthopädische Apparatur lässt sich in zwei unterschiedlichen Ausführungsformen realisieren, nämlich eine erste Ausführungsform, bei der die Plattenabschnitte der Unter- und/oder Oberkieferplatte im Bereich der Schneidezähne unmittelbar ineinander übergehen, und eine zweite Ausführungsform, bei der die Plattenabschnitte voneinander getrennt und über eine zum, bezogen auf die Sagittalebene lateralen, Spreizen bzw. weiter voneinander Beabstanden ausgebildete Dehneinrichtung miteinander verbunden sind. Die Oberkieferplatte umfasst in die Mundhöhle in Richtung Zunge vorstehende Protrusionsmittel, über die sie bei im Kiefer aufgenommener Apparatur mit mindestens einem den Protrusionsmitteln zugeordneten Kontaktbereich zusammenwirkt, derart, dass beim Aufeinanderzubewegen von Ober - und Unterkiefer eine den Unterkiefer nach vorne verstellende Kraft wirkt.

Ferner betrifft die Erfindung ein Verfahren zum Herstellen einer solchen Apparatur gemäß Anspruch 13.

In der Kieferorthopädie werden zum Behandeln von Unterkieferrückstellungen seit Jahrzenten sogenannte Vorschubdoppelplatten eingesetzt, umfassend eine Oberkiefer- und eine Unterkieferplatte, die über oberkieferplattenseitige Protrusionsstäbe aus Metall und diesen zugeordnete unterkieferplattenseitigen Kontaktbereiche zum Verstellen des Unterkiefers beim Schließen des Gebisses zusammenwirken. Dabei tragen die beiden Platten seitliche Drahtschlaufen zum Aufschieben auf die Zähne, meist die Backenzähne, und dadurch Fixieren der Platten in der Mundhöhle. Jede Platte umfasst einen ersten und zweiten, in Bezug auf die Sagittalebene gegenüberliegenden Plattenabschnitt, die einteilig ausgebildet oder über eine Dehneinrichtung miteinander verbunden sein können. Getrennte Plattenabschnitte werden durch Sägen einer zunächst gefertigten Grundplatte erhalten und können daher scharfkantig sein, was eine manuelle Nachbearbeitung, insbesondere Brechen von Kanten und Polieren, etc. notwendig macht. Die metallischen Protrusionsstäbe sind in ein Kunststoffmaterial der Plattenabschnitte der Oberkieferplatte eingebettet und dadurch fixiert. Zur Herstellung wird zunächst ein Negativabdruck des Kiefers manuell hergestellt, aus dem dann manuell ein Kiefermodell erstellt wird, welches dann zur manuellen Formgebung der Platten dient. Dabei wird zunächst die Unterkieferplatte mit ihrem Kontaktbereich (Abgleitfläche) gefertigt. Anschließen wird der Kunststoffteil der Oberkieferplatte gegossen, woraufhin die vorgefertigten, als Fertigteile erhältlichen Protrusionsstäbe positioniert und zur Fixierung mit Kunststoff umgossen werden, wodurch man bei der Fertigung hinsichtlich der Freiheitsgrade der Positionierung und Ausformung der Protrusionsstäbe in engen Grenzen agieren muss und somit nicht jeder Kiefersituation optimal gerecht werden kann. Üblicherweise spannen die Protrusionsstäbe mit der Okklusionsebene einen festen Winkel von 60° oder 65°, wobei je nach Wachstumssituation des Kiefers auch Platten mit einem 55° Winkel eingesetzt werden. Eine solche, bekannte Apparatur ist beispielsweise in der DE 299 15 769 U1 beschrieben.

Als Alternative zur Vorschubdoppelplatte werden, insbesondere im angelsächsischen Raum, zur Korrektur von Unterkieferrücklagen als Twinblock bezeichnete kieferorthopädische Apparaturen eingesetzt, die sich ebenfalls durch einen Materialmix von Metallteilen und Kunststoffteilen auszeichnen.

In jüngerer Zeit ist es zur Herstellung einfacherer kieferorthopädischer Plattensysteme bekannt geworden, 3D-Druck-Verfahren einzusetzen. Hierbei wird die Kiefersituation gescannt und die gewonnen digitalen Daten werden zur Herstellung eines digitalen Kiefermodells genutzt, anhand dessen 3D-Druckdaten zum Drucken der Platten erstellt werden. Hierzu wird beispielshaft auf die US 9,610,141 B2 oder die US 9,795,461 B2 verwiesen.

Auch die Anmelderin entwickelt hochqualitative, 3D-gedruckte und individuell an die jeweilige Kiefersituation angepasste kieferorthopädische Apparaturen aus biokompatiblem Kunststoffmaterial und stellt diese her.

Ausgehend von dem vorgenannten Stand der Technik liegt der Erfindung die Aufgabe zugrunde, eine verbesserte kieferorthopädische Apparatur sowie ein Verfahren zu deren Herstellung anzugeben, die sich durch große Freiheitsgrade bei der Anpassung an die individuelle Kiefersituation auszeichnet, insbesondere durch eine individuelle Ausformbarkeit und Positionierung der Protrusionsmittel und dem/der zugeordnetem/n Kontaktbereich/en, und die trotzdem in der Lage ist, die vergleichsweise großen, auf die Platten wirkenden Kräfte zum Verstellen der Kiefer relativ zueinander zuverlässig und dauerhaft aufzunehmen. Die Apparatur soll zudem für den Patienten komfortabel zu tragen sein und die natürlichen Bewegungsabläufe so wenig wie möglich zu stören.

Diese Aufgabe wird hinsichtlich der kieferorthopädischen Apparatur mit den Merkmalen des Anspruchs 1 und hinsichtlich des Herstellungsverfahrens mit den Merkmalen des Anspruchs 13 gelöst.

Vorteilhafte Weiterbildungen der Erfindung sind in den Unteransprüchen angegeben. Zur Vermeidung von Wiederholungen sollen verfahrensgemäß offenbarte Merkmale auch als vorrichtungsgemäß offenbart gelten und beanspruchbar sein. Ebenso sollen vorrichtungsgemäß offenbarte Merkmale als verfahrensgemäß offenbart gelten und beanspruchbar sein.

Der Erfindung liegt der Gedanke zugrunde, die Protrusionsmittel einteilig mit dem ersten und/oder dem zweiten Oberkieferplattenabschnitt als 3D-Druckteil aus, bevorzugt biokompatiblem, Kunststoff und zudem den mindestens einen Kontaktbereich einteilig mit dem ersten und/oder dem zweiten Unterkieferplattenabschnitt als 3D-Druckteil aus, bevorzugt biokompatiblem, Kunststoff auszubilden. Anders ausgedrückt werden die Protrusionsmittel gemeinsam mit dem ersten und/oder zweiten Oberkieferplattenabschnitt als (ein gemeinsames) 3D - Kunststoffteil gedruckt, also insbesondere übergangslos, bevorzugt im selben Druckvorgang gedruckt, wodurch ein Einbetten oder nachträgliches Fixieren wie im Stand der Technik entfällt. Für den Fall des Vorsehens von voneinander getrennten, über eine Dehneinrichtung miteinander verbundenen, Oberkieferplattenabschnitten ist es bevorzugt, wenn jeder Oberkieferplattenabschnitt Protrusionsmittel aufweist, um eine möglichst symmetrische Krafteinleitung in die Unterkieferplatte zu gewährleisten. In Bezug auf die Unterkieferplatte wird erfindungsgemäß der mindestens eine Kontaktbereich unmittelbar im 3D-Druckverfahren mit dem ersten und/oder zweiten Unterkieferplattenabschnitt gedruckt, also einteilig mit dem ersten und/oder zweiten Unterkieferabschnitt als ein gemeinsames 3D-Druckteil ausgebildet. Bevorzugt sind die Protrusionsmittel mit ihrem von ihrem freien Ende abgewandten distalen an einem oder benachbart zu einem vorderen Gaumenanlageabschnitt des ersten und/oder zweiten Oberkieferplattenabschnittes in einem Bereich (bei bestimmungsgemäßem Einsatz) benachbart der Schneidezähne angeordnet. Die Erfindung schlägt also erstmals ein 3D-gedrucktes Plattensystem vor, welches überraschend in der Lage ist, trotz der Ausbildung der Protrusionsmittel aus Kunststoff dauerhaft und zuverlässig die beim Aufeinanderzubewegen der Kiefer auf die Platten wirkenden Kräfte aufzunehmen. Die erfindungsgemäße Apparatur bietet aufgrund des Einsatzes des 3D-Druckverfahrens und der individuellen Konstruktion/Anpassung an die Kiefersituation in einem CAD-System die Möglichkeit, die Geometrie und Position der Protrusionsmittel sowie des zugeordneten Kontaktbereichs in weiten Grenzen zu variieren und optimal an die individuelle Kiefersituation der Patienten anzupassen. Insbesondere kann, wie später noch erläutert werden wird, die Winkellage der Protrusionsmittel zur bevorzugt einen Bestandteil des digitalen Kiefermodells bildenden Okklusionsebene und/oder zu einem vorderen Gaumenanlageabschnitt der Oberkieferplatte in weiten Grenzen verändert werden, um somit ein optimales, geschmeidiges Schließverhalten und Vorschubverhalten des Unterkiefers sicherzustellen. Hierdurch ist die erfindungsgemäße Apparatur angenehm zutragen und trotz des Verzichts auf metallische Protrusionsmittel dauerhaft stabil und zur Aufnahme der auftretenden Kräfte optimal ausgelegt.

In der Kieferorthopädie wird die Okklusionsebene als Verbindungslinie zwischen zwei speziellen Punkten konstruiert, nämlich zwischen vPOcP: ein konstruierter Punkt, der durch die Halbierung der Strecke des Schneidezahnüberbisses definiert wird, also der Mittelpunkt der Verbindungslinie zwischen den Berührungspunkten der oberen und unteren mittleren Schneidezähne und hPOcP: der distalste Berührungspunkt der letzten in Okklusion stehenden Molaren. Die Okklusionsebene dient der messtechnischen Orientierung für Winkelstellungen / Angulationen der oberen und unteren Zähne zu dieser Okklusionsebene sowie zur Darstellung der Winkelstellung der Okklusionsebene selber zu anderen Bezugslinien des Schädels sowie von kieferorthopädischen Apparaturen.

Die Sagittalebene (auch Medianebene) ist die Körperlängsachsenebene (Mittellinie des Gesichts und des Schädels) und teilt dabei mittig den Körper in etwa zwei gleich große Körperhälften (links und rechts).

Da die erfindungsgemäße Apparatur individuell (patientenspezifisch) anhand eines im Rahmen des erfindungsgemäßen Verfahrens zu erstellenden, dreidimensionalen digitalen Kiefermodells konstruiert und gefertigt wird und die Relativlage von Unterkieferplatte und Oberkieferplatte von der Geometrie des Kiefers und damit des dreidimensionalen digitalen Kiefermodells abhängt und definiert wird, ist es notwendig und zulässig, bestimmte Eigenschaften bzw. Wechselwirkungen und Geometrien der Apparatur anhand von mindestens einer Ebene und/oder Achse des menschlichen Körpers und/oder Kiefers, insbesondere anhand der Relativposition zur Sagittalebene und/oder zur Okklusionsebene. Die Sagittalebene und die Okklusionsebene können (und werden bevorzugt auch) im Rahmen der Erstellung des digitalen Kiefermodells ermittelt werden. Bevorzugt bilden die Okklusionsebene und/oder die Sagittalebene einen Bestandteil des dreidimensionalen digitalen Kiefermodells und ist/sind in diesem darstellbar und/oder dargestellt.

Im Rahmen vorliegender Offenbarung wird unter dem Begriff lateral eine Orientierung senkrecht zur Sagittalebene bzw. senkrecht zu einer von der kieferorthopädischen Apparatur erzwungenen Hauptrelativbewegungsrichtung (Vorwärtsbewegung oder Bewegung von proximal nach distal, parallel zur Sagittalebene) von Unter- zu Oberkiefer beim Schließen des Kiefers durch Wechselwirkung von Protrusionsmitteln mit Kontaktbereich verstanden.

Im Hinblick auf das Herstellungsverfahren liegt der Erfindung der Gedanke zugrunde, zunächst digitale Geometriedaten eines Oberkiefers und eines Unterkiefers zu erfassen, insbesondere durch Scannen mit einem Intraoralscanner, woraufhin ein dreidimensionales digitales Kiefermodell unter Verwendung der digitalen Geometriedaten mit einem CAD-System erstellt wird, wobei es bevorzugt ist, wenn die Sagittalebene und/oder die Okklusionsebene Bestandteil des digitalen Kiefermodells und/oder in diesem darstellbar und/oder dargestellt ist/sind. Anschließend wird dann ein dreidimensionales digitales Modell zumindest der Kunststoffteile der kieferorthopädischen Apparatur, bevorzugt der gesamten Apparatur, an dem Kiefermodell erstellt, wobei das Modell die im Rahmen dieser Offenbarung beschriebenen Merkmale, insbesondere gemäß zumindest eines der Ansprüche 1 bis 12 aufweist (ggf. ohne, jedoch bevorzugt mit, Dehneinrichtung/en, bevorzugt in Form einer vereinfachten dreidimensionalen Darstellung, insbesondere einer äußeren dreidimensionalen Einhüllenden der mindestens einen Dehneinrichtung/en). Die Kunststoffteile der kieferorthopädischen Apparatur werden mit einer 3D-Druckvorrichtung, insbesondere aufgrund der hohen Druckauflösung in einem DLP-Druckverfahren, aus, insbesondere biokompatiblem, Kunststoff gedruckt, derart, dass die Protrusionsmittel einteilig, insbesondere in einem gemeinsamen Druckvorgang, mit dem ersten und/oder dem zweiten Oberkieferplattenabschnitt als 3D-Druckteil aus Kunststoff und der mindestens eine Kontaktbereich einteilig mit dem ersten und/oder dem zweiten Unterkieferplattenabschnitt als 3D-Druckteil aus Kunststoff ausgebildet werden. Anders ausgedrückt wird der 3D-Drucker auf Grundlage des digitalen Apparaturmodells so angesteuert, dass aus dem 3D-Druck Kunststoffteile der Apparatur, insbesondere die Ober- und Unterkieferplattenabschnitte der Apparatur gemäß vorliegender Offenbarung, insbesondere gemäß den Ansprüchen 1 bi 12 resultiert/resultieren, wobei die fakultative mindestens eine Dehneinrichtung bei Bedarf bzw. falls vorgesehen, nach dem Druck an zwei (unteren oder oberen) Plattenabschnitten, insbesondere durch Verkleben in später noch erläuterten plattenabschnittseitigen Öffnungen fixiert wird. Durch die Realisierung der Plattenabschnitte im 3D-Druck ist es möglich und bevorzugt bei einer Ausführungsform der Apparatur mit Dehneinrichtung oder Dehneinrichtungen, die einander zugewandten Kanten der Plattenabschnitte der Ober- und/oder Unterkieferplatte abgerundet zu konstruieren und zu Drucken und damit scharfe Kanten und somit eine manuelle Nachbearbeitung zu vermeiden. Die Protrusionsmittel können dabei, insbesondere im DLP-Druckverfahren aus dem gleichen Material gedruckt werden wie der erste und/oder zweite Oberkieferplattenabschnitt oder, insbesondere mit anderen bekannten Druckverfahren, aus einem davon abweichenden, beispielsweise eine größere Bruchzähigkeit und/oder Biegesteifigkeit aufweisendem Kunststoffmaterial.

Im Rahmen der Konstruktion der erfindungsgemäßen Apparatur, d.h. bevorzugt durch Ausführung des erfindungsgemäßen Verfahrens, insbesondere nach einem der Ansprüche 13 bis 15, wird ein dreidimensionales digitales System erstellt, umfassend das dreidimensionale digitale Kiefermodell, bevorzugt mit Sagittal- und/oder Okklusionsebene, und ein darin angeordnetes dreidimensionales digitales Modell der Kieferorthopädischen Apparatur, insbesondere nach einem der Ansprüche 1 bis 12. Dieses System, in welchem die Relativlage der Unterkieferplatte und der Oberkieferplatte (ggf. ohne Dehneinrichtung/en oder mit einer vereinfachten Darstellung der Dehneinrichtung/en) zueinander und damit zur Okklusions- und/oder Sagittalebene fest definiert sind, soll als erfindungsgemäß offenbart gelten und beanspruchbar sein. Die geometrischen Merkmale des Kiefermodells, im Originalmaßstab oder maßstabsgetreu, entsprechen denen der im Rahmen der Offenbarung beschriebenen kieferorthopädischen Apparatur, insbesondere nach einem der Ansprüche 1 bis 12. Das erfindungsgemäße digitale Modell der Apparatur kann im Rahmen des Systems grundsätzlich ohne eine fakultative Dehneinrichtung erstellt werden, wobei es bevorzugt ist, wenn diese für den Fall, dass die Apparatur mindestens eine solche aufweisen soll, auch Bestandteil des digitalen Modells ist, ggf. wie erläutert, in Form einer vereinfachten Darstellung, insbesondere einer einhüllenden Kontur. Die vereinfachte Darstellung der Dehneinrichtung nimmt die Position der späteren (realen) Dehneinrichtung ein und hilft bei der Positionierung der zugehörigen Aussparungen in den 3D-Druckteilen. Zusätzlich oder alternativ zu dem digitalen Apparaturmodell kann das erfindungsgemäße System gemäß einer Ausführungsvariante die reale kieferorthopädische Apparatur beinhalten, wobei es sich in diesem Fall nicht um ein rein digitales, sondern um ein (erfindungsgemäßes) halbdigitales System handelt.

Die (zweite) Ausführungsform der erfindungsgemäßen Apparatur, bei der die Plattenabschnitte der Ober- und/oder Unterkieferplatte über eine Dehneinrichtung miteinander verbunden sind, um die Plattenabschnitte relativ zueinander lateral zu Verstellen und damit bevorzugt während der Behandlung nach gewissen Zeitabständen weiter voneinander zu beabstanden eignet sich zur Behandlung von Zahnfehlstellungen, d.h. zur Korrektur der Zahnstellung. Anders ausgedrückt lässt sich mit der zweiten Ausführungsform ein lateraler Druck auf Zähne in einander gegenüberliegende Zahnbögenhälften ausüben und damit eine Zahnpositionskorrekur (weitere Indikation für den Einsatz der erfindungsgemäßen Apparatur) vornehmen.

Besonders bevorzugt ist eine Ausführungsform, bei der die seitlichen Taschen zur lösbaren Fixation der Ober- und Unterkieferplatte in der Mundhöhle zumindest teilweise durch Hinterschnitte zum Hintergreifen bzw. Umgreifen von Zahnrundungen begrenzt. Hierdurch lassen sich Ober- und Unterkieferplatte in Zahnzwischenräumen lösbar fixieren, insbesondere einklemmen, wodurch auf die altbekannten metallischen Fixierdrähte verzichtet werden kann und bevorzugt auch verzichtet wird. Ganz besonders bevorzugt ist eine Ausführungsform der Apparatur, bei der die Ober- und Unterkieferplatte dadurch frei von metallischen Fixationsdrähten sind, wobei die Ober- und Unterkieferplatte vollständig metallfrei als 3D-Druckteile aus Kunststoff ausgebildet sind oder für des Fall des Vorsehens von Dehneinrichtungen bis auf diese metallfrei und als 3D-Druckteil ausgebildet sind.

Besonders zweckmäßig ist es, wenn der mindestens eine Kontaktbereich, mindestens einen Begrenzungsvorsprung zum Begrenzen einer Lateralbewegung der Protrusionsmittel beim Entlanggleiten der Protrusionsmittel am Kontaktbereich nach links und/oder rechts, insbesondere in beide Lateralrichtungen aufweist. Bevorzugt handelt es sich um mindestens einen seitlichen Begrenzungsvorsprung, noch weiter bevorzugt zwei lateral beabstandete seitliche Begrenzungsvorsprünge, wobei auch alternative Anordnungen möglich sind. Die Begrenzung der lateralen Relativbewegung in zumindest eine Lateralrichtung kann insbesondere derart realisiert werden, dass der mindestens eine Kontaktbereich als sich von distal nach proximal erstreckende Führungsrinne ausgebildet ist, die die Protrusionsmittel links und rechts führt. Hierdurch können die Kiefer optimaler relativ zueinander positioniert werden und der Bewegungsablauf beim Aufeinanderzubewegen der Kiefer wird vergleichmäßigt. Zudem kann ein lateraler Versatz von Unter- zu Oberkiefer korrigiert werden. Grundsätzlich ist es möglich jedem Protrusionselement eine eigene Führungsrinne oder insbesondere bei Verzicht auf innere Begrenzungsvorsprünge zwei oder mehr Protrusionselemente in einer gemeinsamen Führungsrinne zu führen, die lateral außen jeweils durch einen von zwei beabstandeten und bevorzugt bezogen auf die Sagittalebene gegenüberliegenden Begrenzungsvorsprüngen begrenzt sind. Anstelle von zwei äußeren Begrenzungsvorsprüngen können auch zwei innere Begrenzungsvorsprünge zum Führen von zwei Protrusionselementen an den einander zugewandten Innenseiten vorgesehen werden. Denkbar ist es auch, zumindest oder ausschließlich einem Protrusionselement einen einzigen Begrenzungsvorsprung zum seitlichen Führen des Protrusionselementes an seiner Innen- oder Außenseite zuzuordnen und damit die Bewegung in nur einer Lateralrichtung zu begrenzen. Auch ist es möglich, insbesondere bei einem einzigen, bevorzugt plattenförmigen, Protrusionselement, dieses mit mindestens einem Begrenzungsvorsprung der Unterkieferplatte zu führen, dem eine von distal nach proximal verlaufende Führungsnut an der der Unterkieferplatte zugewandten Seite des Protrusionselementes zugeordnet ist, in der der Begrenzungsvorsprung beim Kieferschließen längsgeführt ist.

Bei Ausführungsformen mit zwei lateral beabstandeten, insbesondere stabförmigen, Protrusionselementen, die jeweils in einer Führungsrinne geführt sind, ist es bei der (zweiten) Ausführungsform mit je einer Dehneinrichtung zwischen den Plattenabschnitten der Ober- sowie Unterkieferplatte bevorzugt, die Dehneinrichtungen während der Behandlung nicht unabhängig voneinander sondern in Abstimmung zu verstellen, derart, dass eine Führung der Protrusionselemente in ihren Führungen gewährleistet bleibt. Bei einer Ausführugsform mit einem einzigen oder mit mehreren plattenförmigen Führungselement(en) ist eine unabhängige Verstellung von Dehneinrichtungen in Ober- und Unterkieferplatte möglich. Auch ist es bei einer solchen Ausführungsform denkbar, enweder nur in der Oberkieferplatte oder nur in der Unterkieferplatte eine Dehneinrichtung vorzusehen und die jeweils andere Platte einteilig zu realisieren. Bei einer unabhängigen Verstellung der Dehneinrichtungen in Ober- und Unterkieferplatte währen der Behandlung oder beim Vorsehen einer Dehneinrichtung ausschließlich in der Ober- oder Unterkieferplatte muss bei der Konstruktion der Platten lediglich der Abstand zwischen äußeren Begrenzungsvorsprüngen so auf die laterale Breite des mindestens einen, bevorzugt plattenförmigen Führungselementes abgestimmt werden (durch Wahl eines entsprechenden Abstandes der Begrenzungsvorsprünge oder Wahl einer entsprechenden Breite des Führungselementes), dass das Führungselement trotz eines Verstellens der Dehneinrichtung noch in seiner Führung bzw. zwischen zwei Begrenzungsvorsprüngen geführt werden kann. Grundsätzlich kann auch bei einer Ausführungsform mit zwei, insbesondere stabförmigen Protrusionselementen die laterale Breite der Führungsrinngen so auf die laterale Breite der Protrusionselemente abgestimmt werden, dass zu Beginn der Behandlung ein größers Führungsspiel akzeptiert wird und nach Betätigen der Dehneinrichtung (weiter Beabstanden von zwei Plattenabschnitten) im Laufe der Behandlung die Protrusionelemente näher in Richtung Rinnenrand bzw. zugehörigem Begrenzungsvorsprung wandern. Daher lässt sich auch eine solche Ausführungsform mit nur einer Dehneinrichtung, entweder in der Ober- oder der Unterkieferplatte, realisieren oder es lassen sich, im Falle des Vorsehens je einer Dehneinrichtung, diese (in Grenzen) unabhängig voneinander verstellen.

Ein Verhaken der Protrusionsmittel und der Unterkieferplatte kann vermieden und der Bewegungsablauf zu Beginn des Schließens des Kiefers optimiert werden, wenn die Begrenzungsvorsprünge in Richtung des mindestens einen freien Endes der Protrusionsmittel orientierte Einlaufschrägen zum Führen der Protrusionsmittel in einen Bereich zwischen den einander gegenüberliegenden Begrenzungsvorsprüngen zu Beginn eines Wechselwirkungsvorgangs zwischen Protrusionsmitteln und Kontaktbereich hinein aufweisen.

Um eine Verletzungsgefahr des Patienten auszuschließen ist es bevorzugt, wenn die Protrusionsmittel ein abgerundetes, insbesondere bogen- oder kugelkalottenförmiges, freies Ende aufweisen. Bevorzugt sind die Protrusionsmittel dabei derart relativ zu dem Kontaktbereich angeordnet, dass ein Erstkontakt beim Aufeinanderzubewegen von Ober- und Unterkiefer zwischen dem freien Ende der Protrusionsmittel und dem Kontaktbereich erfolgt.

Bevorzugt ist es, wenn die Protrusionsmittel auf der von ihrem freien Ende abgewandten Seite in einer, bevorzugt abschnittsweise quaderförmigen, 3D-gedruckten und an der Oberkieferplatte ausgebildeten Blockkonstruktion enden. Hier stützen sich bevorzugt auch fakultative, später noch zu erläuternde Stützstreben ab. Auch ist es bevorzugt, wenn der Kontaktbereich an einer unterkieferplattenseitigen, bevorzugt abschnittsweise quaderförmigen, 3D-gedruckten Blockkonstruktion ausgebildet sind.

Im Hinblick auf die konkrete Ausgestaltung der Protrusionsmittel gibt es unterschiedliche Möglichkeiten. So ist es beispielsweise möglich und bevorzugt, dass die Protrusionsmittel zwei lateral beabstandete, bevorzugt jeweils stab- oder plattenförmige, Protrusionselemente aufweisen, die noch weiter bevorzugt zumindest abschnittsweise, insbesondere vollständig, auf unterschiedlichen Seiten der Sagittalebene angeordnet sind. Alternativ ist es möglich, ein einziges, bevorzugt plattenförmiges, Protrusionselement, vorzusehen, dass sich zu beiden Seiten der Sagittalebene erstreckt und somit eine vergleichsweise große Breite und damit Kraftverteilungsmöglichkeit bietet, Bevorzugt ist es, jedem Protrusionselement ein, insbesondere rinnenförmigen, Kontaktbereich an der Unterkieferplatte zuzuordnen.

Zum Erleichtern der Kieferschließbewegung durch Reibungsminimierung ist es vorteilhaft, wenn die Protrusionsmittel und/oder der Kontaktbereich zwei winklig zueinander angeordnete oder gekrümmt, insbesondere bogenförmig, verlaufende Flächenabschnitte zur Reduzierung der reibwirksamen Kontaktfläche beim Aufeinanderzubewegen von Ober- und Unterkiefer aufweisen/aufweist, wobei alternativ auch die klassische Ausführung mit fluchtenden bzw. in einer Linie liegenden Flächenabschnitten realisierbar ist. Im Falle der Realisierung der winklig oder gekrümmt verlaufenden Flächenabschnitte im Kontaktbereich, ist ein erster vorderer näher in Richtung Schneidezähnen gelegener distaler Flächenabschnitt weniger stark nach unten (also von der Oberkieferplatte weg) geneigt als ein benachbarter, proximal gelegener Flächenabschnitt. Bei geschlossenem Kiefer, also bei maximal in Richtung des Oberkiefers verstelltem Unterkiefer (im digitalen Modell und/oder bei in der Mundhöhle eingesetzter Apparatur) weist bevorzugt ein überstumpfer Winkel ε zwischen dem proximalen und dem distalen Flächenabschnitt des Kontaktbereichs einen Wert zwischen 190° und 260°, weiter bevorzugt zwischen 200° und 255°, ganz besonders bevorzugt zwischen 205° und 245° auf. Zusätzlich oder alternativ ist es bevorzugt, wenn ein bei geschlossenem Kiefer zu messender Winkel γ zwischen dem distalen Flächenabschnitt und der Okklusionsebene aus einem Winkelbereich zwischen 0° und 50°, weiter bevorzugt zwischen 0° und 45°, noch weiter bevorzugt zwischen 1° und 45° gewählt ist. Dabei ist es bevorzugt, wenn die Summe der Winkel ε und γ kleiner ist als 270° und/oder der proximale Flächenabschnitt mit der Okklusionsebene einen Winkel von größer als 0° und kleiner als 90° einschließt. Zusätzlich oder alternativ ist es bevorzugt, wenn zur Reibungsminimierung ein bei geschlossenem Kiefer gemessener ein Winkel δ zwischen dem proximalen Flächenabschnitt des Kontaktbereichs und der Okklusionsebene um einen Wert zwischen 1° und 8°, bevorzugt zwischen 2° und 5° größer ist, also ein später noch zu erläuternder Winkel a, den die Protrusionsmittel mit der Okklusionsebene aufspannen. Gemäß der zuletzt erläuterten bevorzugten Ausführungsform spannen die die Protrusionsmittel und mit dem proximalen Flächenabschnitt also bevorzugt einen Winkel δ - α zwischen 1° und 8°, bevorzugt zwischen 2° und 5° auf. Bevorzugt wirkt ein Kontaktbereich mit winklig oder gekrümmt verlaufenden Flächenabschnitten mit geradlinigen Protrusionsmitteln zusammen, wobei alternativ diese zusätzlich (oder alternativ) gekrümmt oder winklig ausgebildet sein können. Jedenfalls liegen, unabhängig davon, ob nun die Protrusionsmittel und/oder der Kontaktbereich winklig oder gekrümmt zueinander verlaufende (Flächen-)abschnitte aufweist, die Protrusionsmittel bei geschlossenem Kiefer nicht vollständig auf dem Kontaktbereich auf. Bevorzugt sind dabei die Kontaktbereiche und Protrusionsmittel so gestaltet, dass ein erster Kontakt beim Kieferschließen zwischen den Protrusionsmitteln und dem distalen Flächenabschnitt des Kontaktbereichs erfolgt und während des weiteren Kieferschließens die Protrusionsmittel hin zum proximalen Flächenabschnitt des Kontaktbereichs wandern und dann gegen Ende des Schließvorgangs vorzugsweise ausschließlich mit dem proximalen Flächenabschnitt in Kontakt sind bzw. sich an diesem abstützen. Dieser proximale Flächenabschnitt des Kontaktbereichs sollte grundsätzlich bevorzugt mit einem Protrusionsmittelabschnitt bei geschlossenem Kiefer einen Winkel zwischen 1° und 8°, bevorzugt zwischen 2° und 5° aufspannen.

Bei der Ausführungsform mit den winklig zueinander verlaufenden Flächenabschnitten an den Protrusionsmitteln schließt ein distaler Flächenabschnitt mit der Okklusionsebene einen größeren Winkel ein als der flacher verlaufende, proximale, näher am freien Ende der Protrusionsmittel liegende Flächenabschnitt. Ein proximaler (Flächen-)Abschnitt der Protrusionsmittel ist also stärker nach oben (von der Unterkieferplatte weg) geneigt als der vordere, distale (Flächen-)Abschnitt der Protrusionsmittel. Bevorzugt ist der überstumpfe Winkel zwischen den winklig oder gekrümmt zueinander angeordneten Flächenabschnitten aus einem Wertebereich zwischen 190° und 260°, weiter bevorzugt zwischen 200° und 255°, ganz besonders bevorzugt zwischen 205° und 245° gewählt. Bevorzugt beträgt dabei die Summe aus dem vorgenannten Winkel zwischen den Flächenabschnitten der Protrusionsmittel und einem Winkel des distalen Flächenabschnittes zur Okklusionsebene weniger als 270°. Bei vorsehen von winkligen oder gekrümmten Protrusionsmittelabschnitten kommt bevorzugt zunächst ein das freie Ende tragender proximaler Abschnitt der Protrusionsmittel mit dem Kontaktbereich an der Unterkieferplatte in Kontakt und während des weiteren Schließvorgangs hebt bevorzugt dieser proximale Protrusionsmittelabschnitt vom Kontaktbereich ab, so dass vorzugsweise gegen Ende des Schließvorgangs nur noch der distale Protrusionsmittelabschnitt in Kontakt ist mit dem Kontaktbereich bzw. sich an diesem abstützt. Der proximale Flächenabschnitt des Kontaktbereichs sollte bevorzugt mit dem distalen, näher an den Schneidezähnen gelegenen Protrusionsmittelabschnitt bei geschlossenem Kiefer einen Winkel zwischen 1° und 8°, bevorzugt zwischen 2° und 5° aufspannen.

Beide Ausführungsformen (winklige oder gekrümmte Flächenabschnitte im Kontaktbereich und/oder an den Protrusionsmitteln) sind nicht auf die konkret genannten, bevorzugten Wertebereiche für einen Winkelunterschied zur Okklusionsebene beschränkt- wesentlich ist das Erreichen einer Reibungsminimierung im Vergleich zu einer Ausführungsform ohne Winkelunterschied und bevorzugt gleichzeitiger Gewährleistung eines leichtgängigen Kieferschließvorgangs. Unabhängig davon, ob die Flächenabschnitte des Kontaktbereichs winklig oder gekrümmt zueinander verlaufen oder alternativ fluchtend bzw. in einer Linie angeordnet sind und/oder die zur Okklusionsebene geneigten Protrusionsmittel geradlinig ausgebildet sind oder zwei winklig oder gekrümmt zueinander verlaufende Flächenabschnitte aufweisen sind bevorzugt die Okklusionsmittel so auszubilden und anzuordnen und auf die Ausbildung und Anordnung des Kontaktbereichs abzustimmen, dass ab dem Aufeinandertreffen von Protrusionsmitteln und Kontaktbereich bis zum Erreichen einer Kieferschließstellung eine den Unterkiefer nach vorne verstellende Kraft auf den Unterkiefer wirkt bzw. übertragen wird. Insbesondere ist es dazu bevorzugt, wenn ein etwaiger Winkel der Flächenabschnitte des Kontaktbereichs zueinander und zur Okklusionsebene so gewählt und auf die Ausgestaltung und Winkellage der Protrusionsmittel abstimmt wird, dass ab dem Aufeinandertreffen von Protrusionsmitteln und Kontaktbereich bis zum Erreichen einer Kieferschließstellung eine den Unterkiefer nach vorne verstellende Kraft auf den Unterkiefer wirkt bzw. übertragen wird. Ebenso ist vorzugsweise ein etwaiger Winkel von zwei Protrusionsmittelabschnitten zueinander und zur Okklusionsebene so zu wählen und auf die Ausgestaltung und Winkellage des Kontaktbereichs abzustimmen, dass ab dem Aufeinandertreffen von Protrusionsmitteln und Kontaktbereich bis zum Erreichen einer Kieferschließstellung eine den Unterkiefer nach vorne verstellende Kraft auf den Unterkiefer wirkt bzw. übertragen wird.

Besonders bevorzugt ist eine Ausführungsform, bei der die Protrusionsmittel, insbesondere auf der bei bestimmungsgemäßem Einsatz in der Mundhöhle von dem Kontaktbereich der Unterkieferplatte abgewandten Seite, über mindestens eine einteilig im 3D-Druck mit den Protrusionsmitteln sowie dem ersten und/oder zweiten Oberkieferplattenabschnitt ausgebildete Kunststoffstützstrebe an dem ersten und/oder zweiten Oberkieferplattenabschnitt abgestützt sind. Hierdurch wird eine erhöhte Stabilität erreicht und ermöglicht, dass die eigentlichen Protrusionsmittel nicht zu massiv ausgeformt werden müssen. Bevorzugt stützt sich die mindestens eine Kunststoffstrebe an einem vorerwähnten und später noch im Detail erläuterten ersten und/oder zweiten vorderen Gaumenanlageabschnitt benachbart zu den Schneidezähnen ab.

Die erfindungsgemäße Ausgestaltung der Apparatur, insbesondere der Protrusionsmittel und deren einteilige Anbindung (bzw. Herstellung in einem Guss bzw. Druck) an den mindestens einen Oberkieferplattenabschnitt im 3D-Druck ermöglicht die Realisierung einer bevorzugten Ausführungsform, bei der die Protrusionsmittel mit einer bei bestimmungsgemäßem Einsatz in der Mundhöhle zwischen Oberkieferplatte und Unterkieferplatte verlaufenden Okklusionseben einen Winkel α aus einem Bereich zwischen 10° und 50°, bevorzugt zwischen 15° und 45°, noch weiter bevorzugt zwischen 20° und 40° einschließen, wodurch das Schließen des Kiefers mit nur geringem Kraftaufwand für den Patienten zu bewerkstelligen ist. Bei Bedarf können auch größere, insbesondere die klassischen Winkel von 55°, 60° oder 65°, oder auch dazwischen liegende oder noch größere Winkelwerte realisiert werden. So ist es möglich, den Winkel α aus einem Wertebereich zwischen 10° und 65°, bevorzugt zwischen 10° und 60° oder zwischen 10° und 55° zu wählen.

Wie eingangs bereits angedeutet, ist eine Ausführungsform besonders bevorzugt, bei der der erste Oberkieferplattenabschnitt einen mit dem ersten vorderen Gaumenanlageabschnitt zur Anlage im vorderen Gaumenbereich benachbart zu den oberen Schneidezähnen und der zweite Oberkieferplattenabschnitt einen zweiten vorderen Gaumenanlageabschnitt zur Anlage im vorderen Gaumenbereich benachbart zu den oberen Schneidezähnen aufweist, und dass die Protrusionsmittel und der erste und/oder zweite Gaumenanlageabschnitt der Oberkieferplatte, bevorzugt in der Sagittalebene oder einer hierzu parallelen Ebene, einen Winkel aufspannen, bevorzugt aus einem Bereich zwischen 30° und 120°, weiter bevorzugt zwischen 40° und 90°. Das erfindungsgemäße Verfahren ermöglicht es, dass die Protrusionsmittel auch optimal bei unterschiedlichsten Kiefersituationen, also sowohl bei tiefem als auch bei flachem Kiefer positioniert werden können und trotzdem eine optimale Relativposition/Winkel der Protrusionsmittel zur Okklusionsebene und zu dem mindestens einen Kontaktbereich realisiert werden kann. Die beiden Gaumenanlageabschnitte gehen bevorzugt bei einer Ausführungsform ohne Dehneinrichtung nahtlos im Bereich der Sagittalebene ineinander über.

Besonders zweckmäßig ist es für den Fall des Vorsehens der ersten Dehneinrichtung, wenn diese in einander gegenüberliegenden, 3D-gedruckten Oberkieferplattenöffnungen im ersten und zweiten Oberkieferplattenabschnitt mit Klebematerial eingeklebt sind, insbesondere mit UV-härtendem Kunststoffmaterial, weiter bevorzugt mit dem Material der Oberkieferplattenabschnitte identischem Material, wobei das Klebematerial bevorzugt über in die Oberkieferplattenöffnungen mit Randabstand mündende Füllkanäle eingefüllt ist. Zusätzlich oder alternativ ist es für den Fall des Vorsehens der zweiten Dehneinrichtung möglich, diese in einander gegenüberliegenden, 3D-gedruckten Unterkieferplattenöffnungen im ersten und zweiten Unterkieferplattenabschnitt mit Klebematerial eingeklebt sind, insbesondere mit UV-härtendem Kunststoffmaterial, weiter bevorzugt mit dem Material der Unterkieferplattenabschnitte identischem Material, wobei das Klebematerial über in die Unterkieferplattenöffnungen mit Randabstand mündende Füllkanäle eingefüllt ist. Die Dehneinrichtungen können als separate Bauelemente frei auf dem Markt erworben werden. In der Regel handelt es sich um eine Innen-/Außengewindekombination mit gegenläufigen Gewinden. So können beispielsweise in den Öffnungen Innengewindehülsen mit gegenläufigen Innengewinden aufgenommen sein, die über einen Gewindestab mit gegenläufigen Außengewinden verbunden sind. Auch können in den Öffnungen Gewindestäbe mit gegenläufigen Außengewinden aufgenommen werden, die über eine Innengewindehülse mit gegenläufigen Innengewinden verbunden sind.

Um ein Heraushebeln der Oberkieferplatte und/oder der Unterkieferplatte aus der optimalen Sitzposition sicher auch bei extremen Kräften zu verhindern ist eine Ausführungsform bevorzugt, bei der einteilig im 3D-Druck mit dem ersten und/oder zweiten Oberkieferplattenabschnitt ein, bevorzugt rinnenförmiger, bei bestimmungsgemäßem Einsatz die Schneidfläche der oberen Schneidezähne in einem Bereich zwischen den oberen und den unteren Schneidezähnen umgreifender und sich entlang des oberen Zahnbogens, insbesondere, bevorzugt ausschließlich, entlang der Schneidezähne erstreckender, oberer Labialbogen ausgebildet ist und/oder dass einteilig im 3D-Druck mit dem ersten und/oder zweiten Unterkieferplattenabschnitt ein, bevorzugt rinnenförmiger, bei bestimmungsgemäßem Einsatz die Schneidfläche der unteren Schneidezähne in einem Bereich zwischen den oberen und den unteren Schneidezähnen umgreifender und sich entlang des oberen Zahnbogens, insbesondere, bevorzugt ausschließlich, entlang der Schneidezähne, erstreckender, unterer Labialbogen ausgebildet ist. Alternativ ist es möglich, Ober- und Unterkieferplatte labialbogenfrei auszugestalten.

Besonders bevorzugt wird im Rahmen der Herstellung bzw. Konstruktion der erfindungsgemäßen Apparatur die Position der Protrusionsmittel und/oder die Größe der Protrusionsmittel und/oder ein, vorzugsweise in der Sagittalebene oder einer dazu parallelen Ebene liegender, Winkel der Protrusionsmittel zur Okklusionsebene und/oder zu einem vorderen Gaumenanlageabschnitt der Oberkieferplatte kieferspezifisch festgelegt.

Besonders vorteilhaft ist es, wenn der erste und zweite Oberkieferplattenabschnitt einteilig oder als separate Kunststoffteile gedruckt wird/werden, wobei mindestens einer der Oberkieferplattenabschnitte, bevorzugt beide Oberkieferplattenabschnitte, jeweils mit Protrusionsmitteln gedruckt werden und/oder dass der erste und zweite Unterkieferplattenabschnitt einteilig oder als separate Kunststoffteile gedruckt wird/werden, wobei mindestens einer der Unterkieferplattenabschnitte, bevorzugt beide Oberkieferplattenabschnitte, jeweils mit Kontaktbereich gedruckt werden.

Vorteilhafte Ausgestaltungen der Erfindung ergeben sich aus der nachfolgenden Beschreibung bevorzugter Ausführungsbeispiele sowie anhand der Zeichnungen.

Diese zeigen in:
- Fig. 1a - 1e:: ein erstes Ausführungsbeispiel einer nach dem Konzept der Erfindung gestalteten kieferorthopädischen Apparatur mit zwei stabförmigen Protrusionselementen die jeweils mit einem Oberkieferplattenabschnitt einteilig im 3D-Druck hergestellt sind, wobei die Plattenabschnitte relativ lateral verstellbar über eine Dehneinrichtung verbunden sind, wobei lediglich die Unterkieferplatte einen Labialbogen aufweist, wobei alternativ auch eine Ausführung ohne Labialbögen oder nur mit Labialbogen an der Oberkieferplatte oder an beiden Platten realisierbar ist,
- Fig. 2a - 2b:: ein alternatives Ausführungsbeispiel mit jeweils einteiligen Ober- sowie Unterkieferplattenabschnitten ohne Dehneinrichtungen für eine im Vergleich zu dem vorgenannten Ausführungsbeispiel flachere Kiefersituation, beispielhaft mit Labialbögen sowohl an der Ober- als auch an der Unterkieferplatte,
- Fig. 3a-3b:: ein weiteres alternatives Ausführungsbeispiel mit einem einzigen plattenförmigen Protrusionselement und beispielhaft ohne Labialbögen, und
- Fig. 4:: ein weiteres alternatives Ausführungsbeispiel mit zwei plattenförmigen Protrusionsmittel, die auf getrennte Oberkieferplattenabschnitte zu beiden Seiten der Sagittalebene verteilt angeordnet sind.

In den Figuren sind gleiche Elemente und Elemente mit der gleichen Funktion mit den gleichen Bezugszeichen gekennzeichnet.

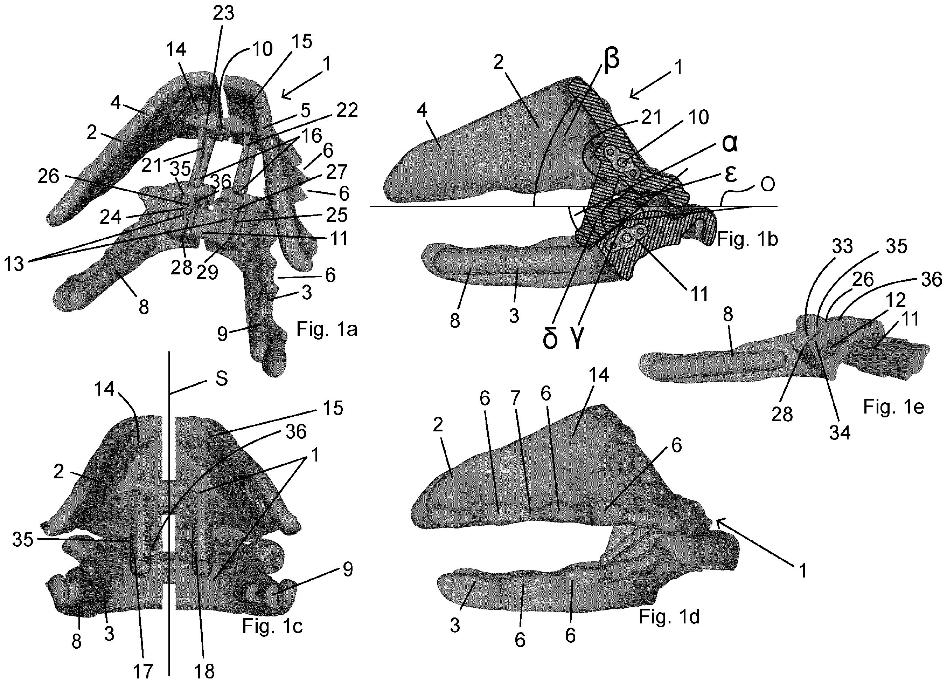

In den Figuren ist mit dem Bezugszeichen 1 eine nach dem Konzept der Erfindung hier als Vorschubdoppelplatte ausgebildete kieferorthopädische Apparatur gekennzeichnet. Diese umfasst eine Oberkieferplatte 2 sowie eine bezogen auf die Okklusionsebene O gegenüberliegende Unterkieferplatte 3. In dem Ausführungsbeispiel gemäß den Fig. 1a bis 1e besteht die Oberkieferplatte 2 aus einem halbbogenförmigen, ersten, linken Oberkieferplattenabschnitt 4 und einem bezogen auf die Sagittalebene S gegenüberliegenden halbbogenförmigen, zweiten, rechten Oberkieferplattenabschnitt 5. Beide Plattenabschnitte 4, 5 ergänzen sich zu einem Bogen und weisen seitliche Taschen 6 zur Anlage an der Mundhöhle bzw. dem Gaumenbereich zugewandten, d.h. palatinalen Zahnflächen auf. Bei bestimmungsgemäßem Einsatz ist der erste Oberkieferplattenabschnitt 4 der linken, oberen Zahnbogenhälfte und der zweite Oberkieferplattenabschnitt 5 der rechten, oberen Zahnbodenhälfte zugeordnet bzw. stützt sich dort ab. Die Taschen sind über endseitige, in Richtung Taschenöffnung gerichtete Hinterschnitte begrenzt, über die Zahnrundungen umgriffen und die Oberkieferplatte an den Zähnen lösbar, insbesondere durch Klemmen, fixiert werden kann. Das gleiche gilt für die Unterkieferplatte 3, die an ihren in Bezug auf die Sagittalebene S gegenüberliegenden Unterkieferplattenabschnitten 8, 9 ebenfalls seitliche Taschen aufweist. Der erste und der zweite Oberkieferplattenabschnitt 3, 4 sind über eine erste Dehneinrichtung 10 Unterkieferplattenabschnitt lateral spreiz- bzw. verstellbar verbunden, ebenso wie der erste und zweite Unterkieferplattenabschnitt 8,9 über eine zweite Dehneinrichtung 11 lateral beabstandbar bzw. verstellbar miteinander verbunden sind.

Wie in Fig. 1e gezeigt ist, ist die zweite Dehneinrichtung 11, ebenso wie die dort nicht gezeigte erste Dehneinrichtung, in einander bezogen auf die Sagittalebene S gegenüberliegenden, 3D-gedruckten lateralen Öffnungen 12 der zugehörigen Plattenabschnitte aufgenommen und dort durch Kleben fixiert. Der Klebstoff wird dabei über seitlich in die Öffnungen 12 mit Öffnungsrandabstand mündende, ebenfalls im 3D-Druck hergestellte Füllkanäle 13 zugeführt. Die Öffnungen befinden sich seitlich in später noch zu erläuternden Blockkonstruktionen 23, 30.

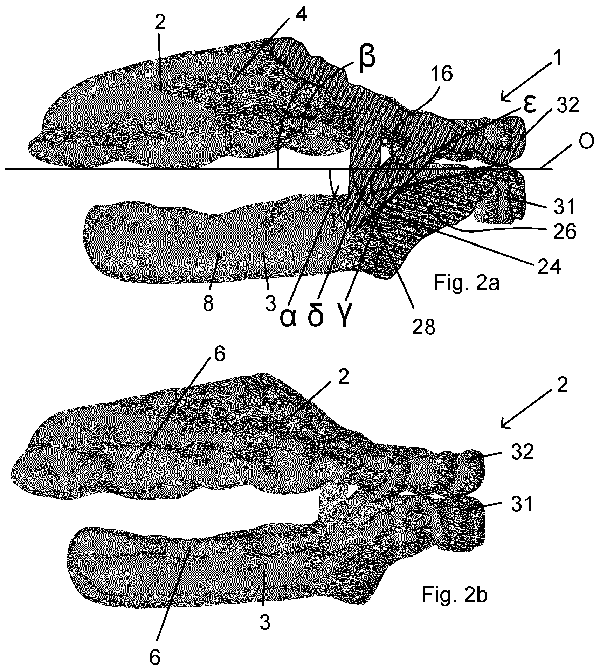

Die Oberkieferplatte 2 und die Unterkieferplatte 3 sind bis auf die Dehneinrichtungen vollständig als 3D-Druckteile hergestellt, vorliegend als jeweils zweiteilige 3D-Druckteile, wobei bei Verzicht auf eine Dehneinrichtung wie in den Fig. 2a - 2b bezeigt ist auch eine einteilige Ausbildung möglich ist. Bis auf die Dehneinrichtungen sind die Oberkieferplatte 2 und die Unterkieferplatte 3 metallfrei und bestehen aus biokompatiblem Kunststoff. Neben dem jeweils halbbogenförmigen Abschnitt der ersten und zweiten Oberkieferplattenabschnitte umfassen diese jeweils einen ersten, linken bzw. zweiten, rechten Gaumenanlageabschnitt 14, 15, die sich ausgehend von einem vorderen Bereich zur Anlage an den Schneidezähnen schräg nach oben erstreckt.

Die jeweils einteilig 3D-gedruckten Oberkieferplattenabschnitte 4,5 umfassen neben dem langgestreckten Bereich mit den Taschen 6 und dem jeweiligen Gaumenanlageabschnitt 14 bzw. 15 Protrusionsmittel 16, die in einem gemeinsamen 3D-Druckprozess als einteiliger bzw. übergangsloser Bestandteil des jeweiligen Oberkieferplattenabschnittes 4,5 verwirklicht sind. In dem Ausführungsbeispiel sind die Kunststoff-Protrusionsmittel 15 in Form von zwei, auf die Oberkieferplattenabschnitte 4,5 verteilt angeordnete, stabförmige Protrusionselemente 17, 18 realisiert, die mit ihren kugelkalottenförmigen freien Enden 19, 20 schräg in Richtung Unterkieferplatte 3 weisen. Die geradlinigen Protrusionselemente 17, 18 enden auf der den freien Enden 19, 20 abgewandten Seiten in einer abschnittsweise quaderförmigen Blockkonstruktion 23, die zumindest Abschnittsweise dem jeweiligen Gaumenanlageabschnitt 14, 15 zugeordnet ist und an der sie sich über von der Unterkieferplatte 3 abgewandte Stützstreben 21, 22 abstützen, die sich entlang der Längserstreckung der Protrusionselemente 17, 18 erstrecken.

Die Protrusionsmittel 16 schließen in einer zur Sagittalebene S parallelen Ebene (hier die durch die Protrusionsmittel 16 verlaufende Schnittebene) einen Winkel α von hier in etwa 40° ein. In derselben Ebene schließen die Protrusionsmittel 16 mit dem jeweils zugehörigen Gaumenanlageabschnitt 14, 15 (allgemein genauer dessen Hocherstreckung) einen Winkel α + β ein, wobei der Winkel β aufgespannt wird von der Okklusionsebene O und dem jeweiligen Gaumenanlageabschnitt 15, 15 bzw. dessen schräger Hocherstreckung. Der Winkel α + β wurde in dem gezeigten Ausführungsbeispiel aufgrund der tiefen Kiefersituation mit etwa 90° gewählt.

Den Protrusionselementen 17, 18 sind an der Unterkieferplatte 3 auf die Unterkieferplattenabschnitte 8, 9 verteilt angeordnete und einteilig im 3D-Druck mit diesen ausgebildete Kontaktbereiche 24, 25 zugeordnet, jeweils umfassend einen distalen Flächenabschnitt 26, 27 und einen jeweiligen distal daran angrenzenden proximalen Flächenabschnitt 28, 29. Die einander zugeordneten Flächenabschnitte 26, 28; 27, 29 verlaufen, wie sich unter anderem aus Fig. 2a und Fig. 1b ergibt, unter einem überstumpfen Winkel ε von hier 210° winklig zueinander, wobei der distale Flächenabschnitt 26, 27 in einer jeweiligen parallel zur Sagittalebene S verlaufenden Ebene mit der Okklusionsebene O (bei geschlossenem Kiefer) einen flacheren Winkel einschließt als der jeweils benachbarte proximale Flächenabschnitt 28, 29. In den Ausführungsbeispielen beträgt ein Winkel γ zwischen dem distalen Flächenabschnitt 26, 27 und der Okklusionsebene etwa 8° und ein Winkel γ zwischen dem proximalen Flächenabschnitt 28, 29 under Okklusionsebene etwa 48°.

Dies führt zu einem reduzierten Berührungs- bzw. Gleitbereich zwischen Protrusionsmitteln 16 und Kontaktbereichen 24, 25 beim Aufeinanderzubewegen der Kiefer und damit der Oberkieferplatte 2 und der Unterkieferplatte 3 aus einer in Fig. 1a gezeigten Öffnungsposition in die in den Fig. 1b - 1d gezeigten Schließposition, in der Protrusionsmittel 16 und Kontaktbereich 24, 25 nicht vollflächig aufeinander aufliegen. Protrusionsmittel 16 und proximaler Flächenabschnitt 28, 29 spannen einen Winkel α-δ von etwa 2° auf. Alternativ kann dies über eine gekrümmte oder winklige Ausbildung der Protrusionsmittel 16 erzielt werden. Zu erkennen ist, dass die Kontaktbereiche 24, 25 an einer unteren, 3D-gedruckten Blockkonstruktion im 3D-Druck ausgeformt sind. Die Kontaktbereiche 24, 25 sind jeweils als Führungsrinne ausgebildet und weist hierzu lateral beabstandete Begrenzungsvorsprünge 33, 34 (lediglich in Fig. 1e angezogen) auf, um eine laterale Relativbewegungsmöglichkeit von Protrusionselementen und Kontaktbereichen 24, 25 zu minimieren. Zur Erleichterten Einführung der Protrusionsmittel 16 in die Kontaktbereich 24, 25 sind die Begrenzungsvorsprünge distal mit Einlaufschrägen versehen.

Den Figuren 1b und 1d ist zu entnehmen, dass hier beispielhaft die unterkieferplatte mit einem Labialbogen 31 ausgestattet ist, der rinnenförmig die Schneidezähne oberhalb der Schneidfläche umgreift und damit in einen Bereich zwischen Unterkieferplatte und Lippen reicht. Der Labialbogen 31 ist auf die beiden Unterkieferplattenabschnitte 8, 9 verteilt angeordnet und jeweils dort einteilig angedruckt.

Das Ausführungsbeispiel gemäß den Fig. 1a bis 1e besteht aus insgesamt vier jeweils einteiligen 3D-Druckteilen in Form der Ober- und Unterkieferplattenabschnitte 4, 5; 8, 9 sowie der Dehneinrichtungen 10, 11.

Im Folgenden werden weitere Ausführungsbeispiele erläutert, wobei zur Vermeidung von Wiederholungen im Wesentlichen auf die Unterschiede zu dem vorhergehenden Ausführungsbeispiel eingegangen wird. Im Hinblick auf die sich offensichtlich für den Fachmann ergebenden Gemeinsamkeiten wird auf die vorstehende Figurenbeschreibung verwiesen.

Das Ausführungsbeispiel einer kieferorthopädischen Apparatur 1 gemäß den Fig. 2a - 2b ist im Vergleich mit dem vorstehenden, ersten Ausführungsbeispiel für eine flachere Kiefersituation ausgelegt, was sich in einem flacheren Winkel β zwischen Protrusionsmitteln 16 und Okklusionsebene O zeigt. Der Winkel α bleibt in dem Ausführungsbeispiel hiervon unbeeinflusst, was der Ausbildung im 3D-Druck geschuldet ist. Im Gegensatz zu dem vorhergehenden Ausführungsbeispiel sind die beiden Oberkieferplattenabschnitte 4, 5 als ein gemeinsames Bauteil aus Kunststoff, also einteilig, gedruckt, da auf eine Dehneinrichtung verzichtet wurde. Die gilt hier auch beispielhaft für die beiden Unterkieferplattenabschnitte 10,11. Die Apparatur 1 besteht also aus nur insgesamt zwei Teilen aus Kunststoff, jeweils ohne Metallteile. Daher kann hier der untere Labialboden 31 auch durchgängig die Sagittalebene A lateral querend ausgebildet werden, ebenso wie ein oberer Labialbogen 32 an der Oberkieferplatte 2.

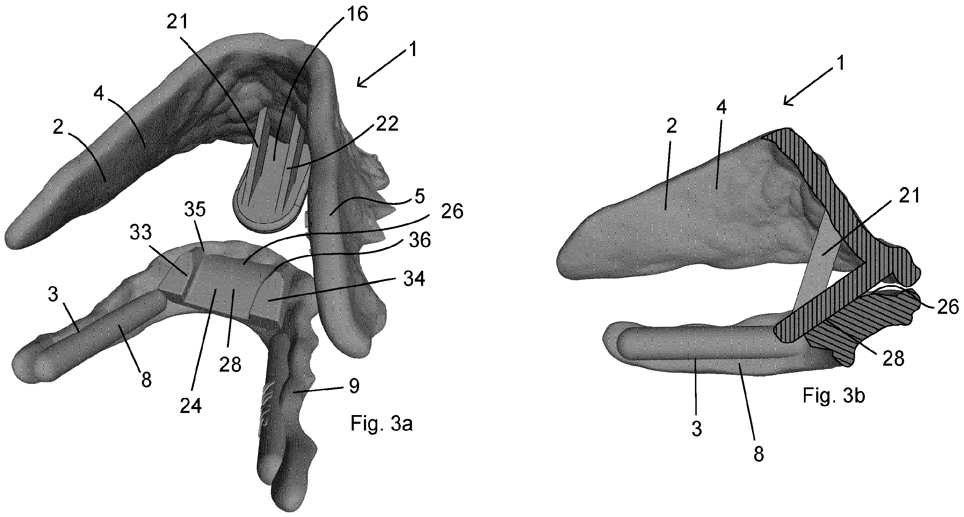

Bei dem Ausführungsbeispiel gemäß den Fig. 3a und 3b sind Ober- und Unterkieferplatte 2, 3 ebenfalls jeweils als einteiliges Kunststoff- 3D-Druckteil ausgebildet, da auf Dehneinrichtungen verzichtet wurde. Die Kiefersituation entspricht in etwa der des Ausführungsbeispiels gemäß den Fig. 1a bis 1f. Die Protrusionsmittel 16 umfassen ein einziges plattenförmiges Protrusionselement 17 mit bogenförmig gerundetem freiem Ende, welches sich durchgehend über die Sagittalebene S erstreckt. Über Stützstreben 21,22 sind die Protrusionsmittel 16 an den einstückigen Oberkieferplattenabschnitten 4, 5 auf einer von der Unterkieferplatte 3 abgewandten Seite abgestützt. Der rinnen- oder kanalförmige Kontaktbereich 24 ist ebenfalls breiter und erstreckt sich lateral über die Sagittaleben S. Seitlich weist der Kontaktbereich 24 Begrenzungsvorsprünge 33, 24 zum Führen der Protrusionsmittel 16 auf, die distal mit die Protrusionsmittel zum Kontaktbereich 24 zentrierenden Einlaufschrägen 35, 36 versehen sind. Wesentlich sind auch hier die winklig zueinander angeordneten Flächenabschnitte 26, 28 zur Begrenzung der Reibungswirksamen Kontaktfläche zwischen Protrusionsmitteln 16 und Kontaktbereich 24.

Insbesondere bei der gezeigten Ausführungsform ist eine zusätzliche oder alternative Begrenzung der Lateralbewegung der Protrusionsmittel möglich, bei der diese eine untere Führungsnut aufweisen, in die ein Begrenzungsvorsprung der Unterkieferplatte 3 eingreift (nicht gezeigt).

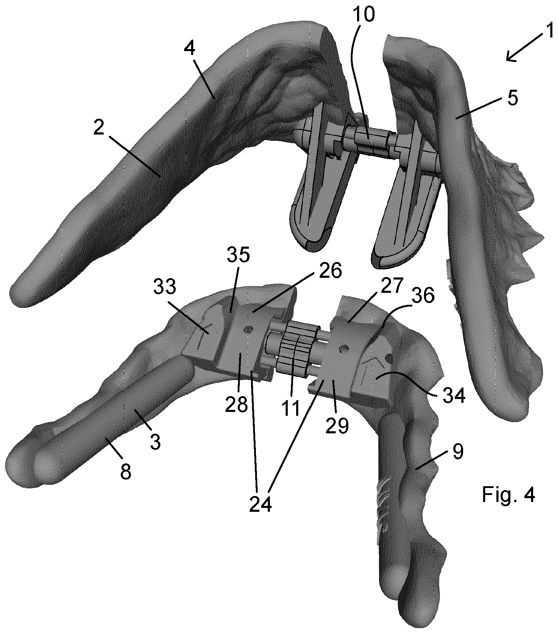

Das Ausführungsbeispiel gemäß Fig. 4 ist im Wesentlichen eine Kombination des ersten und des dritten Ausführungsbeispiels. Die Protrusionselemente 17, 18 sind hier plattenförmig gestaltet. Insgesamt sind lediglich zwei lateral äußere Begrenzungsvorsprünge 33, 34 vorgesehen, was alternativ auch bei dem Ausführungsbeispiel gemäß Fig. 1a bis 1e realisierbar ist. Somit weist der gemeinsame Kontaktbereich 24 für beide Protrusionselemente 17, 18 eine insgesamt führungsrinnenförmige Gestalt auf und umfasst nicht zwei separate Führungsrinnen, da auf die inneren Begrenzungsvorsprünge verzichtet wurde, was allgemein möglich ist.

Insbesondere bei dieser Ausführungsform (analog auch bei der den ersten beiden Ausführungsformen) ist es alternativ möglich, die Protrusionselemente nicht oder nicht nur außen sondern an ihren einander zugewandten Innenseiten mit inneren Begrenzungsvorsprüngen zu führen (nicht gezeigt).

### Bezugszeichenliste

- 1: kieferorthopädische Apparatur
- 2: Oberkieferplatte
- 3: Unterkieferplatte
- 4: erster Oberkieferplattenabschnitt
- 5: zweiter Oberkieferplattenabschnitt
- 6: Taschen
- 7: Hinterschnitte
- 8: erster Unterkieferplattenabschnitt
- 9: zweiter Unterkieferplattenabschnitt
- 10: erste Dehneinrichtung
- 11: zweite Dehneinrichtung
- 12: Öffnungen
- 13: Füllkanäle
- 14: erster Gaumenanlageabschnitt
- 15: zweiter Gaumenanlageabschnitt
- 16: Protrusionsmittel
- 17: Protrusionselement
- 18: Protrusionselement
- 19: freies Ende
- 20: freies Ende
- 21: Stützstrebe
- 22: Stützstrebe
- 23: obere Blockkonstruktion
- 24: Kontaktbereich
- 25: Kontaktbereich
- 26: distaler Flächenabschnitt
- 27: distaler Flächenabschnitt
- 28: proximaler Flächenabschnitt
- 29: proximaler Flächenabschnitt
- 30: untere Blockkonstruktion
- 31: unterer Labialbogen
- 32: oberer Labialbogen
- 33: Begrenzungsvorsprung
- 34: Begrenzungsvorsprung
- 35: Einlaufschräge
- 36: Einlaufschräge

- O: Okklusionsebene
- S: Sagittalebene
- α: Winkel zwischen Protrusionsmitteln und Okklusionsebene
- β: Winkel zw. Gaumenanlageabschnitt und Okklusionsebene
- α + β: Winkel zw. Protrusionsmitteln und Gaumenanlageabschnitt
- γ: Winkel zwischen distalem Flächenabschnitt des Kontaktbereichs und der Okklusionsebene
- δ: Winkel zwischen proximalem Flächenabschnitt des Kontaktbereichs und der Okklusionsebene
- ε: überstumpfer Winkel zwischen proximalem und distalen Flächenabschnitt des Kontaktbereichs
- α - δ: Winkel zwischen Protrusionsmitteln und proximalem Flächenabschnitt des Kontaktbereichs

## Patentansprüche

1. In der Mundhöhle eines Menschen anordenbare kieferorthopädische Apparatur (1) zur Beeinflussung der Relativposition von Ober- und Unterkiefer, insbesondere zur Behandlung von Unterkieferrücklagen, umfassend eine Oberkieferplatte (2) mit einem ersten, insbesondere linken, Oberkieferplattenabschnitt (4) aufweisend seitliche Taschen (6) zur Anlage an palatinalen Zahnflächen eines ersten, insbesondere linken, Zahnbogenabschnittes des Oberkiefers sowie einen zweiten Oberkieferplattenabschnitt (5) mit seitlichen Taschen (6) zur Anlage an palatinalen Zahnflächen eines zweiten, insbesondere rechten, Zahnbogenabschnittes des Oberkiefers und eine Unterkieferplatte (3) mit einem ersten, insbesondere linken, Unterkieferplattenabschnitt (8) aufweisend seitliche Taschen (6) zur Anlage an palatinalen Zahnflächen ersten, insbesondere linken, Zahnbogenabschnitt des Unterkiefers sowie einen zweiten, insbesondere rechten, Unterkieferplattenabschnitt (9) mit seitlichen Taschen (6) zur Anlage an palatinalen Zahnflächen eines zweiten, insbesondere rechten, Zahnbogenabschnittes des Unterkiefers, wobei die Oberkieferplatte (2) Protrusionsmittel (16) zum Zusammenwirken mit einem, bevorzugt mindestens eine schiefe Ebene umfassenden, Kontaktbereich (24, 25) der Unterkieferplatte (3) beim Aufeinanderzubewegen von Ober- und Unterkiefer aufweist, und wobei der erste und der zweite Oberkieferplattenabschnitt (4, 5) entweder einteilig ausgebildet oder über eine erste Dehneinrichtung (10) lateral relativ zueinander verstellbar miteinander verbunden sind und wobei der erste und der zweite Unterkieferplattenabschnitt (8, 9) entweder einteilig ausgebildet oder über eine zweite Dehneinrichtung (11) relativ zueinander verstellbar miteinander verbunden sind,
**dadurch gekennzeichnet,**
**dass** die Protrusionsmittel (16) einteilig mit dem ersten und/oder dem zweiten Oberkieferplattenabschnitt (4, 5) als 3D-Druckteil aus Kunststoff ausgebildet sind und der mindestens eine Kontaktbereich (24, 25) einteilig mit dem ersten und/oder dem zweiten Unterkieferplattenabschnitt (8, 9) als 3D-Druckteil aus Kunststoff ausgebildet ist.

2. Kieferorthopädische Apparatur nach Anspruch 1,
**dadurch gekennzeichnet,**
**dass** die seitlichen Taschen (6) zur lösbaren Fixation der Ober- und Unterkieferplatte (2, 3) in der Mundhöhle zumindest teilweise durch Hinterschnitte (7) zum Hintergreifen von Zahnrundungen begrenzt und die Ober- und Unterkieferplatte (2, 3) dadurch frei von Fixationsdrähten sind, wobei die Ober- und Unterkieferplatte (2, 3) und bevorzugt, ggf. bis auf die fakultativen Dehneinrichtungen (10, 11), metallfrei ausgebildet sind.

3. Kieferorthopädische Apparatur nach einem der vorhergehenden Ansprüche,
**dadurch gekennzeichnet,**
**dass** der, bevorzugt als Führungsrinne ausgebildete, Kontaktbereich (24, 25) mindestens einen Begrenzungsvorsprung (33, 34) zum Begrenzen oder Vermeiden einer Lateralbewegung der Protrusionsmittel (16) beim Entlanggleiten der Protrusionsmittel (16) am Kontaktereich (24, 25) aufweist.

4. Kieferorthopädische Apparatur nach Anspruch 3,
**dadurch gekennzeichnet,**
**dass** mindestens zwei Begrenzungsvorsprünge (33, 34) vorgesehen sind und diese jeweils eine Einlaufschräge (35, 36) zum Führen der Protrusionsmittel (16) in einen Bereich zwischen den einander gegenüberliegenden Begrenzungsvorsprüngen (33, 34) zu Beginn eines Wechselwirkungsvorgangs zwischen Protrusionsmitteln (16) und Kontaktbereich (24, 25) aufweisen.

5. Kieferorthopädische Apparatur nach einem der vorhergehenden Ansprüche,
**dadurch gekennzeichnet,**
**dass** die Protrusionsmittel (16) ein abgerundetes, insbesondere bogenoder kugelkalottenförmiges, freies Ende (19, 20) aufweisen.

6. Kieferorthopädische Apparatur nach einem der vorhergehenden Ansprüche,
**dadurch gekennzeichnet,**
**dass** die Protrusionsmittel (16) zwei lateral beabstandete, bevorzugt jeweils stab- oder plattenförmige, Protrusionselemente (17, 18) aufweisen oder alternativ ein einziges, bevorzugt plattenförmiges, Protrusionselement (17), wobei bevorzugt jedem Protrusionselement (17, 18) ein, insbesondere rinnenförmiger, Kontaktbereich (24, 25) oder ein gemeinsamer, bevorzugt rinnenförmiger, Kontaktbereich (24) an der Unterkieferplatte (3) zugeordnet ist.

7. Kieferorthopädische Apparatur nach einem der vorhergehenden Ansprüche,
**dadurch gekennzeichnet,**
**dass** die Protrusionsmittel (16) und/oder der Kontaktbereich (24, 25) zwei winklig zueinander angeordnete oder gekrümmt, bevorzugt bogenförmig, verlaufende Flächenabschnitte (26, 28; 27, 29) zur Reduzierung der reibwirksamen Kontaktfläche beim Aufeinanderzubewegen von Ober- und Unterkiefer aufweisen/aufweist.

8. Kieferorthopädische Apparatur nach einem der vorhergehenden Ansprüche,
**dadurch gekennzeichnet,**
**dass** die Protrusionsmittel (16), insbesondere auf der bei bestimmungsgemäßem Einsatze in der Mundhöhle von dem Kontaktbereich (24, 25) der Unterkieferplatte (3) abgewandten Seite, über mindestens eine einteilig im 3D-Druck mit den Protrusionsmitteln (16) sowie dem ersten und/oder zweiten Oberkieferplattenabschnitt (4, 5) ausgebildete Kunststoffstützstrebe (21,22) an dem ersten und/oder zweiten Oberkieferplattenabschnitt (4, 5) abgestützt sind.

9. Kieferorthopädische Apparatur nach einem der vorhergehenden Ansprüche,
**dadurch gekennzeichnet,**
**dass** die Protrusionsmittel (16) mit einer bei bestimmungsgemäßem Einsatz in der Mundhöhle zwischen Oberkieferplatte (2) und Unterkieferplatte (3) verlaufenden Okklusionseben (O) einen Winkel (α), bevorzugt aus einem Bereich zwischen 10° und 65°, bevorzugt zwischen 10° und 60°, weiter bevorzugt wischen 10° und 55°, noch weiter bevorzugt zwischen 10° und 50°, noch weiter bevorzugt zwischen 15° und 45°, ganz besonders bevorzugt zwischen 20° und 40° einschließen.

10. Kieferorthopädische Apparatur nach einem der vorhergehenden Ansprüche,
**dadurch gekennzeichnet,**
**dass** der erste Oberkieferplattenabschnitt (4) einen mit dem ersten vorderen Gaumenanlageabschnitt (14) zur Anlage im vorderen Gaumenbereich benachbart zu den oberen Schneidezähnen und der zweite Oberkieferplattenabschnitt (5) einen zweiten vorderen Gaumenanlageabschnitt (15) zur Anlage im vorderen Gaumenbereich benachbart zu den oberen Schneidezähnen aufweist, und dass die Protrusionsmittel (16) und der erste und/oder zweite Gaumenanlageabschnitt (15, 15) der Oberkieferplatte (3), bevorzugt in der Sagittalebene (S) oder einer hierzu parallelen Ebene, einen Winkel (α + β) aufspannen, bevorzugt aus einem Bereich zwischen 30° und 120°, weiter bevorzugt zwischen 40° und 90°.

11. Kieferorthopädische Apparatur nach einem der vorhergehenden Ansprüche,
**dadurch gekennzeichnet,**
**dass** für den Fall des Vorsehens der ersten Dehneinrichtung (10), diese in einander gegenüberliegenden, 3D-gedruckten Oberkieferplattenöffnungen (12) im ersten und zweiten Oberkieferplattenabschnitt mit Klebematerial eingeklebt sind, insbesondere mit UV-härtendem Kunststoffmaterial, weiter bevorzugt mit dem Material der Oberkieferplattenabschnitte (4, 5) identischem Material, wobei das Klebematerial über in die Oberkieferplattenöffnungen (12) mit Randabstand mündende Füllkanäle (13) eingefüllt ist und/oder dass für den Fall des Vorsehens der zweiten Dehneinrichtung (11), diese in einander gegenüberliegenden, 3D-gedruckten Unterkieferplattenöffnungen (12) im ersten und zweiten Unterkieferplattenabschnitt (8, 9) mit Klebematerial eingeklebt sind, insbesondere mit UV-härtendem Kunststoffmaterial, weiter bevorzugt mit dem Material der Unterkieferplattenabschnitte identischem Material, wobei das Klebematerial über in die Unterkieferplattenöffnungen (12) mit Randabstand mündende Füllkanäle (13) eingefüllt ist.

12. Kieferorthopädische Apparatur nach einem der vorhergehenden Ansprüche,
**dadurch gekennzeichnet,**
**dass** einteilig im 3D-Druck mit dem ersten und/oder zweiten Oberkieferplattenabschnitt (4, 5) ein bei bestimmungsgemäßen Einsatz die Schneidfläche der oberen Schneidezähne umgreifender oberer Labialbogen (32) ausgebildet ist und/oder dass einteilig im 3D-Druck mit dem ersten und/oder zweiten Unterkieferplattenabschnitt (8, 9) ein bei bestimmungsgemäßen Einsatz die Schneidfläche der unteren Schneidezähne umgreifender unterer Labialbogen (31) ausgebildet ist, oder dass Ober- und Unterkieferplatte (2, 3) labialbogenfrei sind.

13. Verfahren zum Herstellen einer Kieferorthopädische Apparatur nach einem der vorhergehenden Ansprüche,
**gekennzeichnet durch,**
Erfassen von digitalen Geometriedaten eines Oberkiefers und eines Unterkiefers, insbesondere **durch** Scannen mit einem Intraoralscanner, Erstellen eines dreidimensionalen digitalen Kiefermodells unter Verwendung der digitalen Geometriedaten mit einem CAD-System, Erstellen eines dreidimensionalen digitalen Modells, zumindest der Kunststoffteile, insbesondere der Ober- und Unterkieferplattenabschnitte (4,5; 8,9) der kieferorthopädischen Apparatur (1) an dem Kiefermodell sowie Drucken der Kunststoffteile der kieferorthopädischen Apparatur mit einer 3D-Druckvorrichtung, insbesondere in einem DLP-Druckverfahren, derart, dass die Protrusionsmittel (16) einteilig mit dem ersten und/oder dem zweiten Oberkieferplattenabschnitt (4, 5) als 3D-Druckteil aus Kunststoff und der Kontaktbereich (24, 25) einteilig mit dem ersten und/oder dem zweiten Unterkieferplattenabschnitt (8, 9) als 3D-Druckteil aus Kunststoff gedruckt werden.

14. Verfahren nach Anspruch 13,
**gekennzeichnet durch,**
dass die Position der Protrusionsmittel (16) und/oder die Größe der Protrusionsmittel (16) und/oder ein, vorzugsweise in der Sagittalebene (S) oder einer dazu parallelen Ebene liegender, Winkel (α; α + β) der Protrusionsmittel zur Okklusionsebene (O) und/oder zu einem vorderen Gaumenanlageabschnitt (14, 15) der Oberkieferplatte (3) kieferspezifisch festgelegt werden/wird.

15. Verfahren nach einem der Ansprüche 13 oder 14,
**gekennzeichnet durch,**
dass der erste und zweite Oberkieferplattenabschnitt (4, 5) einteilig oder als separate Kunststoffteile gedruckt wird/werden, wobei mindestens einer der Oberkieferplattenabschnitte (4, 5), bevorzugt beide Oberkieferplattenabschnitte (4, 5), jeweils mit Protrusionsmitteln (16) gedruckt werden und/oder dass der erste und zweite Unterkieferplattenabschnitt (8, 9) einteilig oder als separate Kunststoffteile gedruckt wird/werden, wobei mindestens einer der Unterkieferplattenabschnitte (8, 9), bevorzugt beide Oberkieferplattenabschnitte (8, 9), jeweils mit Kontaktbereich (24, 25) gedruckt werden.
